# EUROPÄISCHE PATENTSCHRIFT

(11) **EP 0 576 853 B1**
(45) Veröffentlichungstag und Bekanntmachung des Hinweises auf die Patenterteilung: **18.10.1995**
(21) Anmeldenummer: 93108909.8
(22) Anmeldetag: 03.06.1993
(51) Int. Cl.: C25B 3/10, C07C 43/12

(54) **Verfahren zur Herstellung von Perfluorpolyethern**
Process for the preparation of perfluoropolyethers
Procédé pour la préparation de perfluoropolyéthers

(30) Priorität: 01.07.1992 DE 4221555
(43) Veröffentlichungstag der Anmeldung: 05.01.1994
(73) Patentinhaber: HOECHST AKTIENGESELLSCHAFT, 65926 Frankfurt am Main (DE)
(72) Erfinder: Jäger, Gerhard, Dr., W-6078 Neu-Isenburg 4 (DE); Millauer, Hans, Dr., W-6236 Eschborn 2 (DE)

(56) Entgegenhaltungen:
- EP-A- 0 355 726
- WO-A-91/15616
- J. ELECTROANAL. CHEM. Bd. 325, Nr. 1-2, 23. M rz 1992, Seiten 167 - 184 V.A. GRINBERG ET AL
- CHEMICAL ABSTRACTS, vol. 113, 1990, Columbus, Ohio, US; abstract no. 86921n, & ZH.PRIKL.KHIM.(Leningrad) Bd.63, Nr. 4, 1990, Seiten 845-849, CH.Z. ESKIBAEVA ET AL

## Beschreibung

Die vorliegende Erfindung betrifft ein Verfahren zur Herstellung von Perfluorpolyethern der allgemeinen Formel 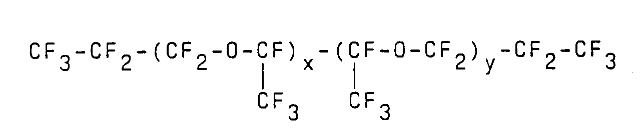
worin x und y für eine ganze Zahl von 1 bis 4 stehen, durch elektrolytische Decarboxylierung von Perfluorcarbonsäuren der allgemeinen Formel 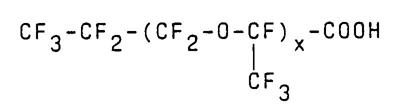
worin x für eine ganze Zahl von 1 bis 4 steht.

Perfluorpolyether besitzen eine ausgezeichnete thermische Stabilität und chemische Beständigkeit, die eine vielseitige Anwendung dieser Verbindungen ermöglichen. Perfluorpolyether können lange Zeit ohne Zersetzung auf ihren Siedepunkt von 200 bis 350°C erhitzt werden. Auch die Anwesenheit organischer Stoffe oder geschmolzener Metalle, wie sie in Dampflötbädern für elektronische Bauteile (vapor phase soldering) eingesetzt werden, führt zu einer kaum merklichen Zersetzung der Perfluorpolyether. Weiterhin können Perfluorpolyether wegen ihrer chemischen Resistenz zum Ausprüfen von elektronischen Komponenten verwendet werden. Dabei ist von Vorteil, daß diese Verbindungen in einem großen Temperaturintervall flüssig sind. Typische Ausprüfungen von elektronischen Bauteilen sind beispielsweise der "Thermal Shock Test", der "Gross Leak Test" und der "Burn-In Test", wie in der EP-A-0 203 348 beschrieben.

Perfluorpolyether zeichnen sich durch eine hohe elektrische Durchschlagsfestigkeit (> 150 kV . cm⁻¹ bei 20°C), sehr niedrige elektrische Leitfähigkeit (< 10⁻⁹ S . cm⁻¹ bei 25°C) und eine kleine Dielektrizitätskonstante (< 2 bei 25°C) aus. Deshalb können Perfluorpolyether zum Kühlen von elektrischen Geräten unter Spannung benutzt werden. Eine weitere besondere Eigenschaft von Perfluorpolyethern ist das ausgezeichnete Lösevermögen für Sauerstoff und Kohlendioxid. Deshalb sind sie als Sauerstoffüberträger in Herz-Lungenmaschinen oder direkt als Blutersatzstoff verwendbar. Ferner können Perfluorpolyether als Sauerstoff- und Kohlendioxidüberträger für abgasfreie Fermentationsprozesse eingesetzt werden.

Perfluorpolyether können als Wärmeüberträgermedien sowie als hydraulische Flüssigkeiten und als Schmier- und Gleitmittel unter extremen chemischen und thermischen Belastungen verwendet werden.

Es ist bereits eine Reihe von Verfahren zur Herstellung von Perfluorpolyethern beschrieben worden.

Die US-A-4,052,277 beschreibt ein photochemisches Verfahren ausgehend von perfluorierten Carbonylverbindungen, welches Belichtungszeiten von 10 bis 20 Stunden erfordert und unter niedriger Raum-Zeit-Ausbeute verläuft.

Einen wirtschaftlicheren Weg für die Herstellung von Perfluorpolyethern bieten die elektrochemischen Kupplungsverfahren, die man als Kolbe-Elektrolysen bezeichnet. Kennzeichnend für die bisher beschriebenen derartigen Herstellverfahren ist die Verwendung von wäßrig-alkoholischen Elektrolyten, in welchen die eingesetzte Perfluorcarbonsäure in Form ihrer Salze oder als Gemische von Salzen und freien Säuren gelöst sind.

In der japanischen Offenlegungsschrift Sho-58-103 334 ist die Herstellung von symmetrischen Perfluorpolyethern mit x = 1 oder 2 und von unsymmetrischen Perfluorpolyethern mit x = 1 und y = 2 beschrieben. Die Perfluorpolyetherausbeuten betragen etwa 37 bis 64 %. Das Verfahren wird in polyalkoholischen Elektrolyten durchgeführt.

In der EP-A-0 355 726 ist die Herstellung von symmetrischen und unsymmetrischen Perfluorpolyethern mit x = 1 bis 4 und y = 1 bis 4 beschrieben. Die Polyfluoretherausbeuten betragen 77 bis 88 % und die Stromausbeuten 52 bis 70 %. Im Verfahren wird als Elektrolyt Methanol oder ein Methanol/Wasser-Gemisch eingesetzt.

In der WO 91/15616 ist die Herstellung von Perfluorpolyethern beschrieben, bei welcher als Elektrolyt ein Wasser/Methanol-Gemisch zum Einsatz kommt. Als besonders vorteilhaft für die Vermeidung einer die Elektrolyse störenden Gel-Bildung wird hier die Verwendung von Kalium-, Ammonium-, Rubidium- oder Caesiumsalzen der Perfluorcarbonsäuren herausgestellt. Die Materialausbeuten erreichen maximal 82,5 %, die Stromausbeuten liegen um 40%.

Die vorstehend beschriebenen Verfahren sind im Hinblick auf die erzielbaren Produktausbeuten nicht befriedigend. Das gilt insbesondere deshalb, weil es sich bei den eingesetzten Perfluorcarbonsäuren um sehr teure Stoffe handelt. Ein weiterer Nachteil sind die niedrigen Stromausbeuten.

Bei der Verwendung von methanolischen Elektrolyten werden Nebenprodukte gemäß folgender Reaktionen gebildet: 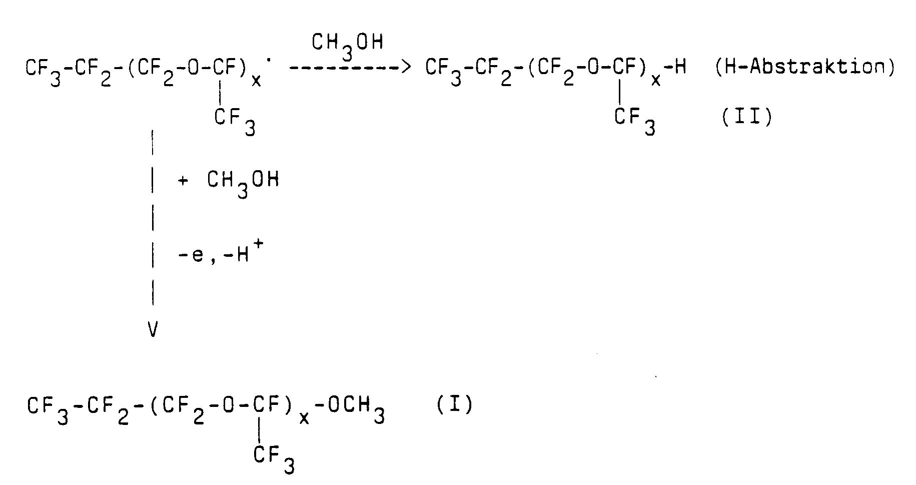
Das bei der Kolbe-Elektrolyse intermediär gebildete Radikal bildet durch Abstraktion von Wasserstoff aus dem Methanol das Nebenprodukt (II). Durch weitere Oxidation des intermediär gebildeten Radikals und anschließende Absättigung mit Methanol entsteht das Nebenprodukt (I) in einer Menge von 3 bis 6 Gewichtsprozenten.

Mit üblichen Reinigungsverfahren wie Extraktion oder fraktionierender Destillation können die Nebenprodukte der Formel (I) nicht oder nur so unvollständig abgetrennt werden, daß die für Perfluorpolyether geforderten hohen Reinheiten von > 99,9 % nicht erreicht werden. Es müssen daher in diesen Fällen zusätzliche, aufwendige Reinigungsoperationen durchgeführt werden. In der DE-A-38 o4 52o ist ein Verfahren zur pyrolytischen Reinigung von Perfluorpolyethern beschrieben, bei welchem der verunreinigte Perfluorpolyether bei 150 bis 360°C dampfförmig über Katalysatoren geleitet wird. Hierbei wird stets auch ein Teil des Perfluorpolyethers vernichtet.

Die Nebenprodukte der Formel (II) werden in Mengen von 1 - 2 % gebildet und erschweren ebenfalls die Reinigung der Perfluorpolyether.

Es war daher die Aufgabe gestellt, ein Verfahren zur Herstellung von Perfluorpolyether anzugeben, bei welchem Perfluorcarbonsäure mit hoher Produkt- und Stromausbeute in Perfluorpolyether umgesetzt und der Perfluorpolyether ohne einen zusätzlichen Reinigungsschritt in einer Reinheit von mehr als 99 % erhalten wird.

Überraschenderweise hat sich gezeigt, daß diese Aufgabe durch eine elektrolytische Decarboxylierung von Perfluorcarbonsäure in wäßriger Elektrolytflüssigkeit in Gegenwart von aliphatischem Nitril mit einem Alkylrest von 1 bis 6 C-Atomen gelöst werden kann.

Das erfindungsgemäße Verfahren kann wahlweise und bevorzugt dadurch gekennzeichnet sein, daß man
1) ein aliphatisches Nitril mit 1 bis 4 C-Atomen verwendet;
2) als aliphatisches Nitril Acetonitril, Propionitril oder Isobutyronitril verwendet;
3) eine wäßrige Elektrolytflüssigkeit mit 1 bis 50, insbesondere 10 bis 30 Gewichtsprozent aliphatischem Nitril einsetzt;
4) eine Elektrolytflüssigkeit mit 1 bis 70, insbesondere 20 bis 50 Gewichtsprozent Perfluorcarbonsäure einsetzt;
5) die elektrolytische Decarboxylierung bei Temperaturen zwischen -10 °C und 90 °C, insbesondere zwischen 0° und 60 °C durchführt;
6) eine Stromdichte von 20 bis 500, insbesondere von 50 bis 250 mA/cm² einstellt;
7) die Perfluorcarbonsäure in Form ihrer Salze oder in teilneutralisierter Form einsetzt, wobei ein Neutralisationsgrad von 5 bis 100 %, vorzugsweise von 50 bis 100 %, eingestellt wird;
8) zur Salzbildung Natrium-, Kalium-, Ammonium- oder Tetraalkylammoniumsalze verwendet;
9) während der elektrolytischen Decarboxylierung Perfluorcarbonsäure in dem Maße nachdosiert, wie diese bei der elektrolytischen Decarboxylierung verbraucht wird.

Bei der Herstellung von symmetrischen Perfluorpolyethern, d.h. die Zahlenwerte für x und y sind identisch, werden Perfluorcarbonsäuren der elektrolytischen Decarboxylierung unterworfen, welche aus identischen Molekülketten aufgebaut sind, d.h. der Zahlenwert für x ist für alle Moleküle gleich groß.

Werden Perfluorcarbonsäuren mit unterschiedlichen Zahlenwerten für x gemeinsam der elektrolytischen Decarboxylierung unterworfen, so ergeben sich asymmetrische Perfluorether im Gemisch mit den symmetrischen Perfluorethern nach folgendem Schema: 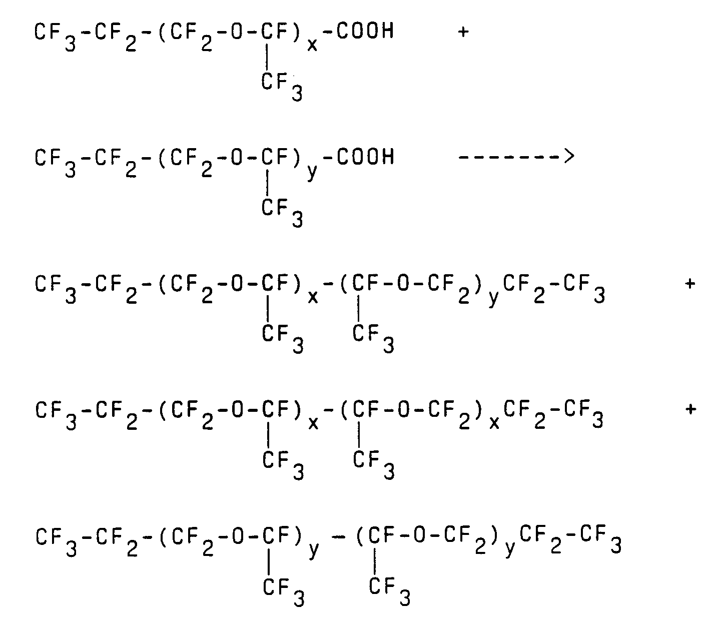
Mit dem erfindungsgemäßen Verfahren werden Produktausbeuten von mehr als 95 bis 98 %, bezogen auf die umgesetzte Perfluorcarbonsäure,erzielt. Bei einem Nitrilanteil von 33 Gewichtsprozent in der Elektrolytflüssigkeit wird eine Stromausbeute von 90 bis 97 % erzielt.

Bei dem erfindungsgemäßen Verfahren ist es nicht erforderlich, den Anteil des eingesetzten Nitrils besonders hoch zu wählen. Während,wie im Vergleichsbeispiel 1 gezeigt wird, bei Abwesenheit von Acetonitril praktisch keine Bildung von Perfluorpolyethern erfolgt, beträgt bei einem Anteil von 4 Gewichtsprozent Acetonitril in der Elektrolytflüssigkeit (Beispiel 2) die Produktausbeute 95,4 % und die Stromausbeute 71,5%. Eine Erhöhung des Acetonitril-Anteils (Beispiele 3 und 4) ergibt nur noch geringfügig erhöhte Ausbeuteverbesserungen.

Bei der Verwendung von Wasser/-Nitril-Gemischen haben die aus der Elektrolytflüssigkeit abtrennbaren Roh-Produkte bereits eine Reinheit von > 99 %, meistens sogar bis zu 99,8 % an Perfluorpolyethern. Das hat eine wesentliche Vereinfachung bei den Reinigungsoperationen zur Folge.

Die Elektrolyse kann im Prinzip in jedem beliebigen zur Elektrolyse geeigneten Behälter durchgeführt werden, beispielsweise in einfachen Becherglaszellen oder in Durchflußzellen.

Normalerweise genügt es, eine ungeteilte Elektrolysezelle zu benützen. Jedoch kann auch eine geteilte Zelle verwendet werden, deren Anolyt- und Katholytraum durch eine Membran oder ein Diaphragma voneinander getrennt sind.

Als Elektroden können die zur Kolbe-Elektrolyse üblichen Materialien zum Einsatz kommen. Als Anode werden Platin oder andere Edelmetalle, mit Platin oder anderen Edelmetallen beschichtete Metalle und glasartiger Kohlenstoff bevorzugt. Als Kathodenmaterial werden Graphit und rostfreie Stähle bevorzugt.

Die nachfolgenden Beispiele sollen das erfindungsgemäße Verfahren näher erläutern.

### Beispiele 1 bis 5

Die verwendete Elektrolysezelle besteht aus einem zylindrischen Glasgefäß (Höhe 150 mm, Innendurchmesser 70 mm), das mit einem Kühlmantel und einem Ablaßhahn am Boden versehen ist. Als Anode dient ein Platinblech (Breite 55 mm, Höhe 110 mm); als Kathode kommt ein grobmaschiges Netz aus rostfreiem Stahl (Breite 55 mm, Höhe 110 mm; Maschenweite 2 mm, Drahtstärke 1 mm) zum Einsatz. Die Elektroden sind parallel zueinander im gegenseitigen Abstand von 5 mm angeordnet und senkrecht an einer Halterung aus Polyethylen am abnehmbaren Deckel der Zelle befestigt. Die in den Elektrolyten eintauchende Elektrodenfläche beträgt ca. 35 cm². Der Elektrolyt wird mittels eines Magnetrührstabes (Länge 55 mm) bewegt.

Man löst jeweils 20,0 g (0,50 Mol) Ätznatron in m Gramm Wasser, gibt dazu unter Umschütteln 246 g ( 0,50 Mol) Perfluor-2,5-dimethyl-3,6-dioxanonansäure [x = 2] und fügt anschließend n Gramm des Nitrils zu. Die Elektrolyse der erhaltenen Lösung erfolgt jeweils bei einer Temperatur von 40°C und einer Stromstärke von 2,70 Ampere bis zu einem Ladungsdurchgang von 21,4 Amperestunden. Die Zellspannung beträgt 5,5 - 6 Volt. Durch Zugabe von weiterer Säure aus einem Tropftrichter wird der pH-Wert der Elektrolytflüssigkeit während der Elektrolyse zwischen 4 und 6 gehalten. Der größte Teil des Rohproduktes trennt sich als schwere Phase von der Elektrolytflüssigkeit ab und kann am Boden der Zelle abgelassen werden. Nach Beendigung der Elektrolyse extrahiert man die bei einem pH-Wert von 7 vorliegende Elektrolytflüssigkeit 3 mal mit je 100 ml 1,1,2-Trichlor-1,2,2-trifluorethan. Anschließend wird die Elektrolytflüssigkeit mit 150 g 50 %iger Schwefelsäure angesäuert und die nicht umgesetzte Perfluorcarbonsäure als schwere Phase abgeschieden und zurückgewonnen. Der Säuregehalt der vorstehend erhaltenen Phase wird durch Titration mit 1 n NaOH bestimmt. Aus der Differenz der Mengen von eingesetzter Säure und zurückgewonnener Säure ergibt sich der Säureverbrauch (p Gramm). Von den vereinigten 1,1,2-Trichlor-1,2,2-trifluorethan-Extrakten wird das Extraktionsmittel abdestilliert, der Rückstand mit der Hauptmenge des Rohprodukts vereinigt und mit 100 ml 1 m Natronlauge extrahiert. Die Menge und die gaschromatographisch ermittelte Zusammensetzung des nach Extraktion mit Natronlauge vorliegenden rohen Perfluorpolyethers sowie die erhaltenen Ausbeuten sind aus der folgenden Tabelle ersichtlich:

| Beispiel Nr. | H₂O m(g) | Nitril n(g) | Säure-Verbrauch p(g) | Perfluorpolyether Rohprodukt | | Produktausbeute (%)^{a)} | Stromausbeute (%) |
|---|---|---|---|---|---|---|---|
| | | | | Menge (g) | Gehalt (%) | | |
| 1^{b)} | 250 | ohne | - | - | - | <1 | <1 |
| 2 | 240 | 10Acetonitril | 298,9 | 258,2 | >99,8 | 95,4 | 71,5 |
| 3 | 235 | 15Acetonitril | 321,2 | 276,0 | >99,8 | 94,4 | 76 |
| 4 | 225 | 25Acetonitril | 324 | 286 | >99,8 | 96,6 | 79 |
| 5 | 240 | 10Propionitril | 238,4 | 197,9 | >99,8 | 91,3 | 55 |

| | | | | | | | |
|---|---|---|---|---|---|---|---|
| ^{a)}die Produktausbeute bezieht sich auf die verbrauchte Säure | | | | | | | |
| ^{b)}Beispiel 1 ist ein nicht erfindungsgemäßes Vergleichsbeispiel | | | | | | | |

### Beispiel 6

Man verwendet eine Elektrolysezelle wie in den Beispielen 1 bis 5 beschrieben; anstelle des Platins wird als Anode eine Platte aus glasartigem Kohlenstoff (Sigradur K®; Breite 55 mm, Höhe 110 mm, Stärke 4 mm) verwendet.
Zu einer Lösung von 10,0 g (0,25 Mol) Ätznatron in 250 g Wasser gibt man unter Umschütteln 165 g (ca. 0,255 Mol) eines Gemisches aus 93 % Perfluor-2,5,8-trimethyl-3,6,9-trioxadodecansäure [x = 3] und 7 % Perfluor-2,5-dimethyl-3,6-dioxanonansäure [x = 2] und fügt zu der erhaltenen Elektrolytflüssigkeit 125 g Acetonitril. Man elektrolysiert bei einer Temperatur von 40°C und einer Stromstärke von 2,7 Ampere bei einer Zellspannung von 7 bis 8 Volt bis zu einer Ladungsmenge von 18,1 Amperestunden. Im Verlauf der Elektrolyse wird durch Zugabe von weiteren 301 g (0,465 Mol) des vorstehend beschriebenen Säuregemisches aus einem Tropftrichter der pH-Wert des Elektrolyten zwischen 4 und 7 gehalten. Der größte Teil des Rohproduktes, etwa 360 g, trennt sich als schwere Phase von der Elektrolytflüssigkeit ab und wird am Boden der Zelle abgelassen. Nach Beendigung der Elektrolyse extrahiert man die bei einem pH-Wert von 7 vorliegende Elektrolytflüssigkeit 3 mal mit je 100 ml 1,1,2-Trichlor-1,2,2-trifluorethan. Der nach dem Abdestillieren des Lösemittels verbleibende Rückstand wird mit der Hauptmenge des Rohprodukts vereinigt und mit 100 ml 1 m Natronlauge gewaschen. Die Menge des erhaltenen Gemisches an rohen Perfluorpolyethern beträgt 367,5 g. Durch Vermischen der Elektrolytflüssigkeit mit 150 g 50 %iger Schwefelsäure und Trennung der Phasen gewinnt man 154 g (0,238 Mol) des als Einsatzstoff verwendeten Perfluorcarbonsäuregemisches zurück, das noch etwas Wasser und Acetonitril enthält.
Die gaschromatographische Analyse des Produktgemisches ergibt einen 99,8 % Anteil an Perfluorpolyethern folgender Zusammensetzung:

| | |
|---|---|
| Perfluorpolyether mit x = 2 | 0,6 Gewichts% |
| Perfluorpolyether mit x = 3 | 83,2 Gewichts% |
| Perfluorpolyether mit x = 2 und y = 3 | 16,0 Gewichts% |

Die Produktausbeute an Perfluorpolyethern beträgt insgesamt 98,3 %, bezogen auf die verbrauchte Säure; die Stromausbeute beträgt 90,5 %.

### Beispiel 7

Man verwendet eine Elektrolysezelle wie in Beispiel 6 beschrieben.

Zu einer Lösung von 8,0 g (0,20 Mol) Ätznatron in 250 g Wasser gibt man unter Umschütteln 166 g (ca. 0,20 Mol) Perfluor-2,5,8,11-tetramethyl-3,6,9,12-tetraoxapentadecansäure [x = 4] und gibt 100 g Propionitril zu. Man elektrolysiert das erhaltene Gemisch bei einer Temperatur von 40°C und einer Stromstärke von 2,7 Ampere bei einer Zellspannung von 9-11 Volt bis zu einer Ladungsmenge von 15,4 Amperestunden. Im Verlauf der Elektrolyse wird durch Zugabe von weiteren 362 g (0,437 Mol) Säure der pH-Wert des Elektrolyten zwischen 4 und 7 gehalten.
Nach Beendigung der Elektrolyse trennt man das Rohprodukt in einem Scheidetrichter von dem Elektrolyten ab und wäscht 2 x mit 1000 ml 1m Natronlauge. Die Menge des rohen Perfluorpolyethers beträgt 312,9 g; die gaschromatographische Analyse des Produktes ergibt einen Anteil von 99,6 % an Perfluorpolyethern folgender Zusammensetzung:

| | |
|---|---|
| Perfluorpolyether mit x = 3 und y = 4 | 0,8 Gewichts% |
| Perfluorpolyether mit x = 4 | 98,8 Gewichts% |

Durch Vermischen des Elektrolyten mit 150 g 50 %iger Schwefelsäure und Separation der erhaltenen Phasen gewinnt man 191,3 g (0,231 Mol) der eingesetzten Säure zurück.

Die Produktausbeute an Perfluorpolyethern beträgt insgesamt 98,0 %, bezogen auf die verbrauchte Säure; die Stromausbeute beträgt 69,3 %.

### Beispiel 8

Es wird eine ungeteilte Durchflußzelle mit parallel angeordneten plattenförmigen Elektroden von je 200 cm² Elektrodenfläche verwendet. Die Anode besteht aus glasartigem Kohlenstoff (Sigradur K®), die Kathode aus Edelstahl. Der Elektrodenabstand beträgt 1 mm.

Der Elektrolytkreislauf besteht aus einer Kreiselpumpe, der vorstehend beschriebenen Zelle und einem Elektrolytsammelgefäß aus Glas, das mit einer Kühlschlange versehen ist. Das Füllvolumen der Apparatur beträgt etwa 2,5 l. An der tiefsten Stelle der Apparatur befindet sich ein Glasstutzen mit einem Ventil zum Ablassen des abgeschiedenen Rohproduktes.

Man löst 80 g (2,0 Mol) Ätznatron in 1000 g Wasser und fügt unter schwachem Umpumpen 1000 g (2,02 Mol) Perfluor-2,5-dimethyl-3,6-dioxanonansäure [x = 2] zu. Die Lösung wird auf einen pH-Wert von 4 - 5 eingestellt und mit 500 g Acetonitril versetzt.

Die Elektrolyseflüssigkeit wird bei einer Temperatur von 40 - 45°C und einer Stromstärke von 16 Ampere bei einer Spannung von 5,3 - 5,5 Volt elektrolysiert. Die Umpumpmenge beträgt etwa 400 l/h. Der pH-Wert der Elektrolyseflüssigkeit wird durch Nachdosieren von weiterer Säure mittels einer pH-gesteuerten Dosierpumpe im Bereich von pH 4 bis 5 gehalten.
Das während der Elektrolyse abgeschiedene Rohprodukt wird in Intervallen abgelassen und gesammelt. Nach 20 Stunden, das entspricht einer Ladungsmenge von 320 Amperestunden, wird die Elektrolyse beendet. Insgesamt werden 5840 g (11,77 Mol) Säure verbraucht und 5247 g Rohprodukt abgeschieden. Das Rohprodukt wird in einen 6 l-Glaskolben mit KPG-Rührer und Bodenablaßhahn überführt und mit 1000 ml 2 n Natronlauge ausgerührt. Nach Trennung der Phasen erhält man 5233 g rohen Perfluorpolyether. Gaschromatographisch wird ein Gehalt von 99,8 % Perfluorpolyether im Rohprodukt bestimmt. Hiervon entfallen 99,3 Gewichts% auf die Verbindung mit x = 2 und 0,5 Gewichts% auf die Verbindung mit x = 2 und y = 1.

Die Produktausbeute an Perfluorpolyethern beträgt 98,5 %, bezogen auf die verbrauchte Säure; die Stromausbeute beträgt 97 %.

Durch Ansäuern des Elektrolyten mit 50 %iger Schwefelsäure kann man die nicht umgesetzte Ausgangssäure zurückgewinnen und für den nächsten Elektrolyseansatz wiederverwenden. Die zur Extraktion benutzte Natronlauge läßt sich ebenfalls für den Elektrolyten des folgenden Ansatzes einsetzen.

### Beispiel 9

Es wird eine Elektrolysevorrichtung, wie in Beispiel 8 beschrieben, verwendet.
Man löst 80 g (2,0 Mol) Ätznatron in 1000 g Wasser und fügt dazu unter schwachem Umpumpen ein Gemisch aus 330 g (1,0 Mol) Perfluor-2-methyl-3-oxahexansäure [x = 1] und 662 g (1,0 Mol) Perfluor-2,5,8-trimethyl-3,6,9-trioxadodecansäure [x = 3]; die erhaltene Lösung wird auf einen pH-Wert von 4 - 5 eingestellt und mit 500 g Acetonitril versetzt.

Die Elektrolyseflüssigkeit wird bei einer Temperatur von 40 - 45°C und einer Stromstärke von 16 Ampere bei einer Spannung von 5,3 - 5,4 Volt elektrolysiert. Die Umpumpmenge beträgt etwa 400 l/h. Der pH-Wert der Elektrolyseflüssigkeit wird durch Nachdosieren von weiterem Säuregemisch der oben angegebenen Zusammensetzung mittels einer pH-gesteuerten Dosierpumpe im Bereich von 4,5 bis 5,5 gehalten. Das während der Elektrolyse abgeschiedene Rohprodukt wird in Intervallen abgelassen und gesammelt. Nach 10 Stunden, entsprechend einer Ladungsmenge von 160 Amperestunden,wird die Elektrolyse beendet. Insgesamt wurden 2648 g Rohprodukt abgeschieden. Insgesamt wurden 2720 g (5,48 Mol) Säure verbraucht. Das Rohprodukt wird in einen Rührkolben mit Bodenablaßhahn überführt und mit 1000 ml 2 n Natronlauge ausgerührt. Nach Trennung der Phasen erhält man 2553 g (5,335 Mol) rohen Perfluorpolyether. Das gaschromatographisch analysierte Produktgemisch besteht zu 99,7 % aus Perfluorpolyethern der folgenden Zusammensetzung:

| | |
|---|---|
| Perfluorpolyether mit x = 1 | 10,0 Gewichts% |
| Perfluorpolyether mit y = 3 | 41,6 Gewichts% |
| Perfluorpolyether mit x = 1 und y = 2 | 0,2 Gewichts% |
| Perfluorpolyether mit x = 1 und y = 3 | 47,5 Gewichts% |
| Perfluorpolyether mit x = 2 und y = 3 | 0,4 Gewichts% |

Die Produktausbeute an Perfluorpolyethern beträgt insgesamt 97,3 %, bezogen auf die verbrauchte Säure; die Stromausbeute beträgt 89,4 %.

### Beispiel 1o

Man verwendet eine Elektrolysezelle wie in Beispiel 6 beschrieben. Zu einer Lösung von 2o g (o,5o Mol) Ätznatron in 225 g Wasser fügt man unter Umschütteln 165 g (o,5o Mol) Perfluor-2-methyl-3-oxahexansäure (x = 1) und anschließend 25 g Isobutyronitril zu. Die erhaltene Lösung wird bei einer Temperatur von 4o °C und einer Stromstärke von 2,7 Ampere bei einer Zellspannung von 7 Volt elektrolysiert. Im Verlauf der Elektrolyse wird durch Zugabe von 255 g Ausgangssäure der pH-Wert zwischen 4 und 7 gehalten. Die gesamte Ladungsmenge beträgt 23,2 Amperestunden.
Aus dem Elektrolyten scheiden sich 166,7 g Rohprodukt ab; durch dreimalige Extraktion des Elektrolyten mit je 1oo ml 1,1,2-Trichlor-1,2,2-trifluorethan und Abdestillieren des Extraktionsmittels erhält man weitere 6o,5 g Rohprodukt.
Nach Waschen der vereinigten Rohprodukte mit 1oo ml 1 n Natronlauge verbleiben insgesamt 226 g Produkt mit einem gaschromatographisch ermittelten Anteil von 99,4 % an Perfluorpolyether mit x = 1.
Durch Ansäuern des Elektrolyten mit Schwefelsäure, mehrmalige Extraktion mit 1,1,2-Trichlor-1,2,2-trifluorethan und anschließendem Verdampfen des Extraktionsmittels werden 14o g Ausgangssäure zurückgewonnen.
Die Produktausbeute an Perfluorpolyether beträgt 92,5 %, bezogen auf die verbrauchte Säure; die Stromausbeute beträgt 91 %.

## Patentansprüche

1. Verfahren zur Herstellung von Perfluorpolyethern der allgemeinen Formel 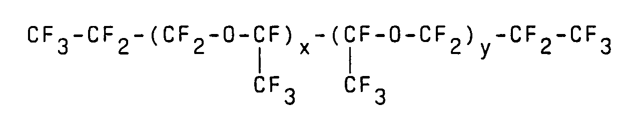 worin x und y für eine ganze Zahl von 1 bis 4 stehen, durch elektrolytische Decarboxylierung von Perfluorcarbonsäure der allgemeinen Formel 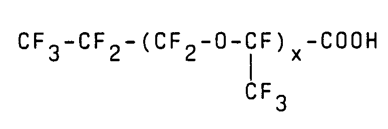 worin x für eine ganze Zahl von 1 bis 4 steht, dadurch gekennzeichnet, daß man die elektrolytische Decarboxylierung in wäßriger Elektrolytflüssigkeit in Gegenwart von aliphatischem Nitril mit einem Alkylrest von 1 bis 6 C-Atomen durchführt.

2. Verfahren nach Anspruch 1, dadurch gekennzeichnet, daß man ein aliphatisches Nitril mit 1 bis 4 C-Atomen verwendet.

3. Verfahren nach einem der Ansprüche 1 und 2, dadurch gekennzeichnet, daß man als aliphatisches Nitril Acetonitril, Propionitril oder Isobutyronitril verwendet.

4. Verfahren nach einem der Ansprüche 1 bis 3, dadurch gekennzeichnet, daß man eine wäßrige Elektrolytflüssigkeit mit 1 bis 50, insbesondere 10 bis 30 Gewichtsprozent aliphatischem Nitril einsetzt.

5. Verfahren nach einem der Ansprüche 1 bis 4, dadurch gekennzeichnet, daß man eine Elektrolytflüssigkeit mit 1 bis 70, insbesondere 20 bis 50 Gewichtsprozent Perfluorcarbonsäure einsetzt.

6. Verfahren nach einem der Ansprüche 1 bis 5, dadurch gekennzeichnet, daß man die elektrolytische Decarboxylierung bei Temperaturen zwischen -10°C und 90°C, insbesondere zwischen 0° und 60°C durchführt.

7. Verfahren nach einem der Ansprüche 1 bis 6, dadurch gekennzeichnet, daß man eine Stromdichte von 20 bis 500, insbesondere von 50 bis 250 mA/cm² einstellt.

8. Verfahren nach einem der Ansprüche 1 bis 7, dadurch gekennzeichnet, daß man die Perfluorcarbonsäure in Form ihrer Salze oder in teilneutralisierter Form einsetzt, wobei ein Neutralisationsgrad von 5 bis 100 %, vorzugsweise von 50 bis 100 %, eingestellt wird.

9. Verfahren nach einem der Ansprüche 1 bis 8, dadurch gekennzeichnet, daß man zur Salzbildung Natrium-, Kalium-, Ammonium- oder Tetraalkylammoniumsalze verwendet.

10. Verfahren nach einem der Ansprüche 1 bis 9, dadurch gekennzeichnet, daß man während der elektrolytischen Decarboxylierung Perfluorcarbonsäure in dem Maße nachdosiert, wie diese bei der elektrolytischen Decarboxylierung verbraucht wird.

## Claims

1. A process for preparing perfluoropolyethers of the formula 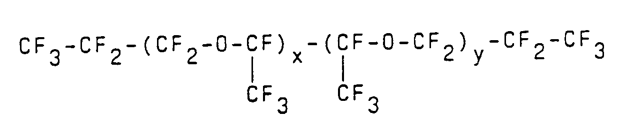 in which x and y are an integer from 1 to 4, by electrolytic decarboxylation of perfluorocarboxylic acid of the formula 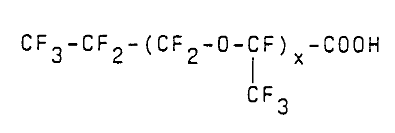 in which x is an integer from 1 to 4, which comprises carrying out the electrolytic decarboxylation in an aqueous electrolyte fluid in the presence of aliphatic nitrile having an alkyl radical of from 1 to 6 carbon atoms.

2. The process as claimed in claim 1, wherein an aliphatic nitrile having from 1 to 4 carbon atoms is used.

3. The process as claimed in any one of claims 1 and 2, wherein the aliphatic nitrile used is acetonitrile, propionitrile or isobutyronitrile.

4. The process as claimed in any one of claims 1 to 3, wherein an aqueous electrolyte fluid is used comprising from 1 to 50, in particular from 10 to 30, percent by weight of aliphatic nitrile.

5. The process as claimed in any one of claims 1 to 4, wherein an electrolyte fluid is used comprising from 1 to 70, in particular from 20 to 50, percent by weight of perfluorocarboxylic acid.

6. The process as claimed in any one of claims 1 to 5, wherein the electrolytic decarboxylation is carried out at temperatures between -10°C and 90°C, in particular between 0° and 60°C.

7. The process as claimed in any one of claims 1 to 6, wherein a current density of from 20 to 500, in particular from 50 to 250, mA/cm² is set.

8. The process as claimed in any one of claims 1 to 7, wherein the perfluorocarboxylic acid is used in the form of its salts or in part-neutralized form, a degree of neutralization of from 5 to 100 %, preferably from 50 to 100 % being set.

9. The process as claimed in any one of claims 1 to 8, wherein salts of sodium, potassium, ammonium or tetraalkylammonium are used for salt formation.

10. The process as claimed in any one of claims 1 to 9, wherein during the electrolytic decarboxylation, more perfluorocarboxylic acid is metered in in proportion to the extent to which it is consumed in the process of electrolytic decarboxylation.

## Revendications

1. Procédé de préparation de perfluoropolyéthers de formule générale : 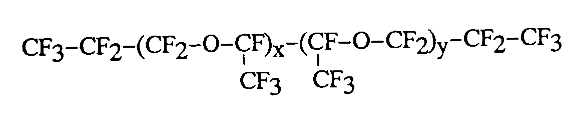 dans laquelle x et y représentent chacun un nombre entier allant de 1 à 4, par décarboxylation électrolytique d'acides perfluorocarboxyliques de formule générale : 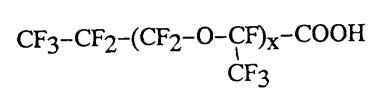 dans laquelle x est un nombre entier allant de 1 à 4, caractérisé en ce que l'on procède à la décarboxylation électrolytique dans un électrolyte liquide aqueux en présence d'un nitrile aliphatique dont le groupe alkyle est en C₁-C₆.

2. Procédé selon la revendication 1, caractérisé en ce que l'on utilise un nitrile aliphatique en C₁-C₄.

3. Procédé selon une des revendications 1 et 2, caractérisé en ce que le nitrile aliphatique utilisé est l'acétonitrile, le propionitrile ou l'isobutyronitrile.

4. Procédé selon une des revendications 1 à 3, caractérisé en ce que l'on utilise un électrolyte liquide aqueux contenant 1 à 50, plus spécialement 10 à 30 % en poids de nitrile aliphatique.

5. Procédé selon une des revendications 1 à 4, caractérisé en ce que l'on utilise un électrolyte liquide contenant 1 à 70, plus spécialement 20 à 50 % en poids d'acide perfluorocarboxylique.

6. Procédé selon une des revendications 1 à 5, caractérisé en ce que l'on procède à la décarboxylation électrolytique à des températures allant de - 10 à + 90°C, plus spécialement de 0 à 60°C.

7. Procédé selon une des revendications 1 à 6, caractérisé en ce que l'on règle la densité de courant à un niveau de 20 à 500, plus spécialement de 50 à 250 mA/cm².

8. Procédé selon une des revendications 1 à 7, caractérisé en ce que l'on met en oeuvre l'acide perfluorocarboxylique à l'état de sel ou à l'état partiellement neutralisé, à un taux de neutralisation de 5 à 100 %, de préférence de 50 à 100 %.

9. Procédé selon une des revendications 1 à 8, caractérisé en ce que, pour la formation du sel, on utilise un sel de sodium, de potassium, d'ammonium ou de tétraalkylammonium.

10. Procédé selon une des revendications 1 à 9, caractérisé en ce que, au cours de la décarboxylation électrolytique, on ajoute des compléments de l'acide perfluorocarboxylique au fur et à mesure de sa consommation par la décarboxylation électrolytique.
